# EUROPÄISCHE PATENTANMELDUNG

(11) **EP 3 569 278 A1**
(43) Veröffentlichungstag der Anmeldung: **20.11.2019**
(21) Anmeldenummer: 19170862.7
(22) Anmeldetag: 24.04.2019
(51) Int. Cl.: A61M 25/00

(54) **KATHETERANORDNUNG UND VERFAHREN ZUR HERSTELLUNG EINER SOLCHEN KATHETERANORDNUNG**

(30) Priorität: 16.05.2018 DE 102018207642
(71) Anmelder: B. Braun Melsungen AG, 34212 Melsungen (DE)
(72) Erfinder: Dubielzig, Egbert, 34560 Fritzlar (DE); Kahlen, Oliver, 34281 Gudensberg (DE)
(74) Vertreter: Patentanwälte Ruff, Wilhelm, Beier, Dauster & Partner mbB

(57) **Zusammenfassung**

Katheteranordnung (1) mit einem Katheterschlauch (2) mit einem Lumen (6, 7), wobei das Lumen ein proximales Ende (8, 9) und ein distales Ende (10, 11) aufweist, und wenigstens einer Zuleitung (4, 5), wobei die Zuleitung ein distales Ende (14, 15) und ein proximales Ende (16, 17) aufweist, und wobei das distale Ende der Zuleitung fluidleitend mit dem proximalen Ende des Lumens verbunden ist., ist bekannt. Die fluidleitende Verbindung zwischen der Zuleitung und dem Lumen ist mittels einer fluiddichten Fügeverbindung zwischen dem distalen Ende der Zuleitung und dem proximalen Ende des Lumens bewirkt.

## Beschreibung

Die Erfindung betrifft eine Katheteranordnung mit einem Katheterschlauch mit wenigstens einem Lumen, wobei das Lumen ein proximales Ende und ein distales Ende aufweist, und wenigstens einer Zuleitung , wobei die Zuleitung ein distales Ende und ein proximales Ende aufweist, und wobei das distale Ende der Zuleitung fluidleitend mit dem proximalen Ende des Lumens verbunden ist.

Die Erfindung betrifft zudem ein Verfahren zur Herstellung einer solchen Katheteranordnung.

Eine derartige Katheteranordnung ist im Bereich der Medizintechnik allgemein bekannt und in Form eines mehrlumigen Venenkatheters zur Verwendung bei einer Infusions- und/oder einer Transfusionstherapie vorgesehen. Die bekannte Katheteranordnung weist einen Katheterschlauch auf, durch welchen ein erstes Lumen und ein zweites Lumen erstreckt sind, die jeweils ein distales Ende und ein proximales Ende aufweisen. Die distalen Enden der Lumen sind in einem Abschnitt des Katheterschlauchs angeordnet, der in einem bestimmungsgemäß applizierten Zustand der Katheteranordnung in beispielsweise eine Vene eines Patienten eingeführt ist. Demgegenüber sind die proximalen Enden der Lumen in appliziertem Zustand der Katheteranordnung außerhalb des Patientenkörpers angeordnet. Weiter sind eine erste Zuleitung und eine zweite Zuleitung mit jeweils einem proximalen Ende und einem distalen Ende vorgesehen. Die erste Zuleitung ist dem ersten Lumen zugeordnet. Die zweite Zuleitung ist dem zweiten Lumen zugeordnet. Die proximalen Enden der Zuleitungen können jeweils auf grundsätzlich bekannte Weise mit einem Anschlusselement zur fluidleitenden Verbindung mit einem Infusionsbehälter oder dergleichen versehen sein. Die distalen Enden der Zuleitungen sind fluidleitend mit dem proximalen Ende des jeweils zugeordneten Lumens verbunden. Auf diese Weise werden zwei voneinander getrennte Kanäle gebildet. Diese Kanäle erstrecken sich ausgehend vom proximalen Ende der jeweiligen Zuleitung bis hin zu dem distalen Ende des der jeweiligen Zuleitung zugeordneten Lumens. Die derart voneinander getrennten Kanäle der Katheteranordnung ermöglichen beispielsweise eine simultane Verabreichung inkompatibler medizinischer Flüssigkeiten, wobei eine Vermischung der Flüssigkeiten innerhalb der Katheteranordnung vermieden wird.

Die bekannte Katheteranordnung weist zudem eine als Kunststoffspritzgussbauteil gefertigte Verbindungseinheit zur fluidleitenden Verbindung der Zuleitungen mit dem jeweiligen Lumen auf. Diese Verbindungseinheit wird auch als Kanaltrennung bezeichnet. Die Verbindungseinheit ist zwischen den distalen Enden der Zuleitungen und den proximalen Enden der Lumen angeordnet und sowohl an die Zuleitungen als auch an den Katheterschlauch angespritzt. Die Verbindungseinheit weist zwei nebeneinander angeordnete und voneinander getrennt erstreckte Verbindungskanäle auf. Die distalen Enden der Zuleitungen sowie die proximalen Enden der Lumen münden an gegenüberliegenden Stirnendseiten der Verbindungseinheit in die Verbindungskanäle. Die Verbindungskanäle bilden insoweit jeweils einen fluidführenden Abschnitt der voneinander getrennten Kanäle der Katheteranordnung.

Zur Herstellung der bekannten Katheteranordnung wird jeweils ein Stützelement in Form einer sogenannten Umspritzungsnadel in das proximale Ende des ersten Lumens bzw. das proximale Ende des zweiten Lumens eingeführt. Weiter werden die Zuleitungen an ihrem jeweiligen distalen Ende auf die Umspritzungsnadel des jeweils zugeordneten Lumens aufgeschoben, wobei die distalen Enden der Zuleitungen und die proximalen Enden der Lumen voneinander beabstandet sind. Weiter wird die derartige Anordnung in ein Spritzgusswerkzeug eingelegt und im Bereich der Umspritzungsnadeln mit einem thermoplastischen Kunststoff umspritzt. Hiernach werden die Umspritzungsnadeln demontiert und die derart gebildete Verbindungseinheit wird aus dem Spritzgusswerkzeug entfernt. Die Umspritzungsnadeln dienen zum einen der Stützung der Lumen und der Zuleitungen, da diese ansonsten während des Kunststoffspritzgießens druckbedingt kollabieren würden. Zum anderen bilden die Umspritzungsnadeln Negativformen für den jeweiligen Verbindungskanal, der sich zwischen dem distalen Ende der Zuleitung und dem proximalen Ende des zugeordneten Lumens erstreckt.

Bei der Herstellung der Verbindungseinheit mittels Kunststoffspritzgießens können Fertigungsfehler auftreten, beispielsweise Lunker, Spannungsrisse oder eine unvollständige Ausformung. Infolgedessen kann es zu Undichtigkeiten der fluidleitenden Verbindungen zwischen den distalen Enden der Zuleitungen und den proximalen Enden der Lumen oder einer unerwünschten fluidleitenden Querverbindung zwischen den Verbindungskanälen der Verbindungseinheit kommen.

Aufgabe der Erfindung ist es, eine Katheteranordnung der eingangs genannten Art sowie ein Verfahren zur Herstellung einer solchen zu schaffen, die gegenüber dem Stand der Technik verbesserte Eigenschaften aufweisen.

Diese Aufgabe wird für die Katheteranordnung dadurch gelöst, dass die fluidleitenden Verbindung zwischen der Zuleitung und dem Lumen mittels einer fluiddichten Fügeverbindung zwischen dem distalen Ende der Zuleitung und dem proximalen Ende des Lumens bewirkt ist. Durch die erfindungsgemäße Lösung wird eine unmittelbare fluidleitende Verbindung zwischen der Zuleitung und dem Lumen erreicht. Demzufolge kann auf eine ansonsten übliche, zusätzliche Verbindungseinheit verzichtet werden. Die mit einer solchen Verbindungseinheit einhergehenden Nachteile und Risiken, insbesondere die durch eine mangelhafte Fertigung bedingte Ausbildung von Undichtigkeiten oder Querverbindungen zwischen den Verbindungskanälen, können insoweit vermieden werden. Die fluiddichte Fügeverbindung kann insbesondere als Press-, Steck-, Schraub-, Kleb- und/oder Schweißverbindung zwischen dem distalen Ende der Zuleitung und dem proximalen Ende des Lumens ausgebildet sein. Beispielsweise kann das distale Ende der Zuleitung in das proximale Ende des zugeordneten Lumens eingesteckt, -gepresst oder -geschraubt sein oder umgekehrt. Alternativ oder zusätzlich kann das distale Ende der Zuleitung mit dem proximalen Ende des Lumens verklebt und/oder verschweißt sein. Vorzugsweise ist die fluiddichte Fügeverbindung unlösbar. Im Sinne der Erfindung bezeichnet "proximal" eine von einer Katheterspitze der Katheteranordnung abgewandte Lage. Demgegenüber bezeichnet "distal" eine der Katheterspitze zugewandte Lage.

Die erfindungsgemäße Lösung eignet sich in besonders bevorzugter Weise für eine mehrlumige Katheteranordnung mit zwei oder mehr Lumen und dementsprechend zwei oder mehr Zuleitungen, die jeweils einem der Lumen zugeordnet sind. Die erfindungsgemäße Lösung ist jedoch nicht auf solche mehrlumige Katheteranordnungen beschränkt, sondern auch bei einer einlumigen Katheteranordnung mit lediglich einem Lumen und einer Zuleitung anwendbar.

In Ausgestaltung der Erfindung ist das distale Ende der Zuleitung in das proximale Ende des Lumens, insbesondere fluiddicht, eingesteckt. Soweit zwei Lumen und zwei Zuleitungen vorgesehen sind, ist eine erste Steckverbindung zwischen der ersten Zuleitung und dem ersten Lumen und/oder eine zweite Steckverbindung zwischen der zweiten Zuleitung und dem zweiten Lumen gebildet. Diese können als solche fluiddicht ausgebildet sein, beispielsweise mittels einer entsprechenden Anpassung der Außendurchmesser der Zuleitungen an die Innendurchmesser der Lumen, so dass sich jeweils eine fluiddichte Presspassung ergibt. Alternativ können die Steckverbindungen nicht oder nur eingeschränkt fluiddicht ausgebildet sein, wobei die distalen Enden der Zuleitungen mit dem proximalen Ende des jeweiligen Lumens fluiddicht verschweißt und/oder verklebt sein können zur Ausbildung der Fluiddichtigkeit der Fügeverbindung. Entsprechendes gilt, sofern lediglich eine Steckverbindung bei einer einlumigen Katheteranordnung vorgesehen ist.

In weiterer Ausgestaltung der Erfindung weist das distale Ende der Zuleitung einen stirnendseitig angeformten Einführabschnitt auf, der komplementär zu einem stirnendseitig angeformten Aufnahmeabschnitt des proximalen Endes des Lumens ausgebildet ist. Der Einführabschnitt der Zuleitung ist stirnendseitig an das distale Ende angeformt und kann insbesondere als Zapfen, Bund oder Nippel ausgebildet sein. Der Aufnahmeabschnitt ist am proximalen Stirnende des Lumens angeformt und kann insbesondere nach Art einer Muffe oder mittels einer radialen Aufweitung des Lumens ausgebildet sein. Vorzugweise ist der Einführabschnitt einstückig an die Zuleitung angeformt. Demgemäß ist es vorteilhaft, wenn der Aufnahmeabschnitt einstückig an den Katheterschlauch bzw. an das Lumen angeformt ist.

In weiterer Ausgestaltung der Erfindung ist der Aufnahmeabschnitt gegenüber einem Strömungsquerschnitt des Lumens, insbesondere thermisch, aufgeweitet. Unter einem Strömungsquerschnitt des Lumens ist ein Querschnitt zu verstehen, der zur Fluidführung vorgesehen ist. Der Strömungsquerschnitt kann insbesondere rund, oval, kreisförmig, halbkreis- oder sichelförmig sein. Der Aufnahmeabschnitt kann insbesondere mittels Einpressens eines geeigneten Werkzeugs in die proximalen Enden des Lumens ausgebildet sein. Sofern eine mehrlumige Katheteranordnung mit wenigstens zwei Lumen und wenigstens zwei Zuleitungen vorgesehen ist, können die Strömungsquerschnitte und somit die Aufnahmeabschnitte unterschiedlich geformt sein.

In weiterer Ausgestaltung der Erfindung ist die Fügeverbindung, insbesondere fluiddicht, verschweißt. Demzufolge weist die Fügeverbindung zwischen dem distalen Ende der Zuleitung und dem proximalen Ende des Lumens eine Schweißverbindung auf. Die Schweißverbindung kann als solche fluiddicht ausgeführt sein. Beispielsweise kann das distale Ende der Zuleitungen "stumpf" mit dem proximalen Ende des Lumens verschweißt sein. Alternativ kann die fluiddichte Fügeverbindung eine Steckverbindung nach den vorstehenden Ausführungen aufweisen, wobei die Schweißverbindung zusätzlich vorgesehen ist. Sowohl die Zuleitung als auch der Katheterschlauch sind wenigstens im Bereich der Fügeverbindung vorzugsweise aus Kunststoff gefertigt. Hierbei ist die Kunststoffzusammensetzung der Zuleitungen derart auf die Kunststoffzusammensetzung des Katheterschlauchs abgestimmt, dass diese mittels grundsätzlich bekannter Kunststoffschweißverfahren zusammengefügt werden können.

In weiterer Ausgestaltung der Erfindung ist eine Kapseleinheit vorgesehen, die als Niederdruck-Spritzgussbauteil aus Kunststoff gefertigt und derart gestaltet und/oder angeordnet ist, dass der Bereich der fluiddichten Fügeverbindung zwischen der Zuleitung und den Lumen mittels der Kapseleinheit verkapselt ist. Als Niederdruck-Spritzgussbauteil gefertigt meint, dass die Kapseleinheit unter Anwendung eines bislang vornehmlich im Bereich der Elektrotechnik bekannten Fertigungsverfahrens, das auch als Low-Pressure-Molding bezeichnet wird, hergestellt ist. Die Kapseleinheit umschließt die fluiddichten Fügeverbindung zwischen der Zuleitung und dem Lumen gleichsam einer Umhüllung, einer Ummantelung oder eines Gehäuses. Vorzugsweise weist die Kapseleinheit einends einen konisch ausgeformten Anlageabschnitt auf, der in bestimmungsgemäß appliziertem Zustand der Katheteranordnung zur Anlage an einer patientenseitigen Eintrittsstelle des Katheterschlauchs vorgesehen ist. Zudem ist es vorteilhaft, wenn die Kapseleinheit Fixierabschnitte aufweist, mittels derer die Kapseleinheit und damit die Katheteranordnung patientenseitig auf grundsätzlich bekannte Weise befestigt werden kann.

In weiterer Ausgestaltung der Erfindung bewirkt die Kapseleinheit eine zusätzliche Fluidabdichtung und/oder eine Zugkraftentlastung der fluiddichten Fügeverbindung. Die mittels der Kapseleinheit bewirkte Fluidabdichtung ist insoweit redundant. Bei einem ungewollten Lösen der fluiddichten Fügeverbindung zwischen dem distalen Ende der Zuleitung und dem proximalen Ende des Lumens kann demzufolge eine Undichtigkeit der Katheteranordnung vermieden werden. Durch diese Ausgestaltung der Erfindung wird demnach eine besonders zuverlässige Abdichtung der Verbindung zwischen der Zuleitung und dem Katheterschlauch erreicht. Soweit die Kapseleinheit als Niederdruck-Spritzgussbauteil gefertigt ist, ist diese fest, insbesondere stoffschlüssig, mit der Zuleitung und mit dem Katheterschlauch verbunden. Auf diese Weise wird eine Zugkraftentlastung der fluiddichten Fügeverbindung erreicht. Demzufolge kann einem ungewollten Lösen der fluiddichten Fügeverbindung mittels der Kapseleinheit entgegengewirkt werden. Dies ist eine besonders robuste und patientensichere Ausgestaltung der Erfindung.

In weiterer Ausgestaltung der Erfindung ist die Kapseleinheit aus Schmelzklebstoff, insbesondere aus Polyamid oder Polyolefin, gefertigt. Schmelzklebstoffe sind als solche grundsätzlich bekannt und weisen eine thermoplastische Zusammensetzung auf. Schmelzklebstoffe sind zur Verarbeitung mittels Niederdruck-Spritzgießens bevorzugt geeignet, da sie im schmelzflüssigen Zustand eine vergleichsweise geringe Viskosität aufweisen. Hierdurch bedingt kann eine Verarbeitung bei - im Vergleich zu herkömmlichem Kunststoffspritzgießen - niedrigen Drücken erfolgen. Auf diese Weise kann eine druckbedingte Deformation der fluiddichten Fügeverbindung bei der Ausbildung der Kapseleinheit vermieden werden.

In weiterer Ausgestaltung der Erfindung ist die Katheteranordnung eine Zentralvenenkatheteranordnung oder eine peripher eingeführte Katheteranordnung (PICC-Line) oder eine Midline-Kathetheranordnung.

Die der Erfindung zugrunde liegende Aufgabe wird für ein Verfahren der eingangs genannten Art dadurch gelöst, dass ein Schritt vorgesehen ist: fluiddichtes Zusammenfügen des distalen Endes der Zuleitung mit dem proximalen Ende des Lumens. Durch die erfindungsgemäße Lösung kann auf eine aufwändige und potentiell fehleranfällige Herstellung der fluiddichten Verbindung mittels einer ansonsten üblichen Verbindungseinheit verzichtet werden. Stattdessen wird das distale Ende der Zuleitung, vorzugsweise unmittelbar, mit dem proximalen Ende des Lumens zusammengefügt, beispielsweise mittels einer Press-, Steck-, Schraub-, Kleb- und/oder Schweißverbindung. Dementsprechend wird zur Herstellung einer mehrlumigen Katheteranordnung das distale Ende der jeweiligen Zuleitung fluiddicht mit dem proximalen Ende des jeweiligen Lumens zusammengefügt.

In weiterer Ausgestaltung der Erfindung weist das Verfahrenen einen Schritt auf: Anformen eines stirnendseitigen Einführabschnitts an das distale Ende der Zuleitung. Das Anformen des Einführabschnittes erfolgt vorzugsweise mittels Umformens des distalen Endes der Zuleitung. Hierbei wird der Einführabschnitt vorzugsweise komplementär zu einer Querschnittsform des Lumens angeformt, beispielsweise rund, oval, kreis- oder halbkreisförmig, halbmond- oder sichelförmig. Das derartige Anformen des Einführabschnittes ermöglicht ein vereinfachtes und passgenaues Zusammenfügen der Zuleitung mit dem Lumen.

In weiterer Ausgestaltung der Erfindung weist das Verfahren einen Schritt auf: Anformen eines stirnendseitigen Aufnahmeabschnitts an das proximale Ende des Lumens mittels radialen Aufweitens des Lumens. Der Aufnahmeabschnitt wird vorzugsweise mittels Umformens an das proximale Ende des Lumens angeformt. Beispielsweise kann ein entsprechend gestaltetes Werkzeug in Form eines Dorns, eines Stifts oder dergleichen in das proximale Ende des Lumens eingepresst werden, so dass dieses plastisch radial aufgeweitet wird. Vorzugsweise wird der Aufnahmeabschnitt im Wesentlichen konisch ausgeformt. Sofern die Zuleitung mit einem entsprechenden Einführabschnitt versehen ist, ist es vorteilhaft, wenn die Gestalt des Aufnahmeabschnittes derart auf die Gestalt des Einführabschnittes abgestimmt ist, dass sich zwischen diesen eine Übergangs- oder Presspassung ergibt.

In weiterer Ausgestaltung der Erfindung erfolgt das Anformen des Einführabschnittes und/oder des Aufnahmeabschnittes mittels Umformens unter Druck- und/oder Wärmeeinwirkung. Sowohl die Zuleitung als auch der Katheterschlauch sind vorzugsweise aus thermoplastischem Kunststoff gefertigt. Thermoplastische Kunststoffe erweichen unter Wärmeeinwirkung und sind in erweichtem Zustand unter Aufwendung vergleichsweise geringer Druck- bzw. Umformkräfte umformbar. Diese Ausgestaltung der Erfindung erlaubt eine besonders einfache und kostengünstige Herstellbarkeit der Einführ- und/oder Aufnahmeabschnitte.

In weiterer Ausgestaltung der Erfindung wird bei dem fluiddichten Zusammenfügen der Einführabschnitt der Zuleitung in den Aufnahmeabschnitt des Lumens eingesteckt. Demzufolge wird eine unmittelbare kraft- und/oder formschlüssige Verbindung zwischen den distalen Enden der Zuleitung und dem proximalen Ende des Lumens hergestellt. Hierbei kann die Fluiddichtigkeit der Fügeverbindung bereits durch das Einstecken des Einführabschnittes in den Aufnahmeabschnitt als solches bewirkt werden. Beispielsweise indem die Außenkontur des Einführabschnittes derart auf die Innenkontur des Aufnahmeabschnittes abgestimmt ist, dass sich eine Presspassung zwischen der Zuleitung und dem jeweiligen Lumen und somit eine Fluiddichtigkeit ergibt. Alternativ oder zusätzlich kann die Fluiddichtigkeit der Fügeverbindung nach dem Einstecken des Einführabschnittes in den Aufnahmeabschnitt mittels weiterer Verfahrensschritte, beispielsweise einem Verkleben, einem Verschweißen oder dergleichen bewirkt werden.

In weiterer Ausgestaltung der Erfindung wird bei dem fluiddichten Zusammenfügen das distale Ende der Zuleitung mit dem proximalen Ende des Lumens, insbesondere der Einführabschnitt mit dem Aufnahmeabschnitt, verschweißt. Soweit die Zuleitung und der Katheterschlauch aus thermoplastischem Kunststoff gefertigt sind, kann das distale Ende der Zuleitung mit dem proximalen Ende des Lumens mittels grundsätzlich bekannter Kunststoffschweißverfahren, beispielsweise mittels Zirkular-, Laserdurchstrahl-, Induktions- oder Ultraschallschweißens miteinander verbunden werden.

In weiterer Ausgestaltung der Erfindung weist das Verfahrenen einen Schritt auf: Prüfen, insbesondere mittels computergestützter Bilderkennung und Bildverarbeitung, einer Zuordnung des Einführabschnittes zu dem Aufnahmeabschnitt und/oder einer Zuordnung de distalen Endes der Zuleitung zu den proximalen Enden des Lumens. Um eine Erfassung und/oder Identifikation der jeweiligen Abschnitte und/oder Enden mittels computergestützter Bildverarbeitung gewährleisten zu können, ist es vorteilhaft, wenn die Abschnitte und/oder die Enden mit entsprechenden Markierungselementen, beispielsweise einer farbigen und/oder grafischen Markierung, versehen sind. Prüfen mittels computergestützter Bildverarbeitung meint, dass zur Vermeidung einer fehlerhaften Zuordnung der Abschnitte und/oder Enden und damit zur Qualitätssicherung ein im Bereich der Automatisierungstechnik als solches grundsätzlich bekanntes Verfahrens, das auch als maschinelles Sehen oder Bildverstehen bezeichnet wird, verwendet wird. Das derartige Prüfen der Zuordnung der Abschnitte und/oder der Enden kann insbesondere bei der Herstellung einer mehrlumigen Katheteranordnung beispielsweise vor dem Einstecken der Einführabschnitte in den jeweiligen Aufnahmeabschnitt erfolgen. Alternativ oder zusätzlich kann das Prüfen der Zuordnung vor dem Verschweißen der Zuleitungen mit dem Katheterschlauch bzw. der Einführabschnitte mit dem jeweiligen Aufnahmeabschnitt erfolgen. Zudem ist es vorteilhaft, wenn bei dem Prüfen zudem die Dichtigkeit der Fügeverbindung zwischen dem distalen Ende der jeweiligen Zuleitung und dem proximalen Ende des jeweiligen Lumens überprüft wird. Zu diesem Zweck kann die computergestützte Bilderkennung und Bildverarbeitung derart eingerichtet sein, dass ein Mangel an der Fügeverbindung, beispielsweise eine unvollständig ausgeführte Schweißverbindung, detektierbar ist.

In weiterer Ausgestaltung der Erfindung weist das Verfahren einen Schritt auf: Verkapseln der fluiddichten Fügeverbindung zwischen der Zuleitung und den Lumen mittels Niederdruck-Kunststoffspritzgießens, wobei eine Kapseleinheit ausgebildet wird. Niederdruck-Kunststoffspritzgießen ist insbesondere im Bereich der Herstellung elektrischer und/oder elektronischer Komponenten grundsätzlich bekannt. Es wird auch als Low-Pressure-Molding bezeichnet. Bei diesem Verfahren wird ein thermoplastischer Schmelzklebstoff, beispielsweise Polyamid oder Polyolefin, unter Wärmeeinwirkung verflüssigt. Beispielsweise wird der Schmelzklebstoff auf eine Temperatur von 180 °C bis 240 °C, vorzugsweise 210 °C erwärmt. Der derart verflüssigte Schmelzklebstoff wird bei vergleichsweise geringem Druck, vorzugsweise im Bereich von 3,5 bar bis 14 bar, in eine entsprechende Spritzgussform gepresst. Aufgrund der derart niedrigen Spritzdrücke eignet sich Low-Pressure-Molding vorzugsweise zur Verkapselung empfindlicher Bauteile, die unter Einwirkung höherer Drücke, wie sie beispielsweise bei einem konventionellen Kunststoffspritzgießen vorherrschen, deformiert und/oder beschädigt werden könnten. Auf eine ansonsten übliche Stützung des Lumens und der Zuleitung mittels einer sogenanntes Umspritzungsnadel kann demzufolge verzichtet werden. Durch diese Ausgestaltung der Erfindung kann insbesondere eine verbesserte Abdichtung der fluiddichten Fügeverbindung sowie eine Zugkraftentlastung zwischen der Zuleitung und dem Katheterschlauch erreicht werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Darstellung eine Ausführungsform einer erfindungsgemäßen Katheteranordnung mit einem Katheterschlauch und zwei Katheterzuleitungen ,
- Fig. 2: in einer schematischen Querschnittdarstellung den Katheterschlauch der Katheteranordnung entlang einer Schnittlinie A-A gemäß Fig. 1,
- Fig. 3: in einer schematischen Längsschnittdarstellung die Katheteranordnung nach den Fig. 1 und 2 im Bereich einer fluiddichten Fügeverbindung zwischen den Zuleitungen und den Lumen des Katheterschlauchs,
- Fig. 4: in einer schematischen Perspektivdarstellung einen proximalen Bereich des Katheterschlauchs der Katheteranordnung nach den Fig. 1 bis 3,
- Fig. 5: in einer schematischen Perspektivdarstellung einen distalen Bereich einer der Zuleitungen der Katheteranordnung nach den Fig. 1 bis 4,
- Fig. 6: in einer schematischen Flussdarstellung eine Ausführungsform eines erfindungsgemäßen Verfahrens zur Herstellung der Katheteranordnung nach den Fig. 1 bis 5,
- Fig. 7: in einer schematischen Flussdarstellung Details eines Verfahrensschrittes des Verfahrens nach Fig. 6,
- Fig. 8: in einer schematischen Flussdarstellung Details eines weiteren Verfahrensschritts des Verfahrens nach Fig. 6 und
- Fig. 9: in einer schematischen Flussdarstellung Details eines weiteren Verfahrensschritts des Verfahrens nach Fig. 6.

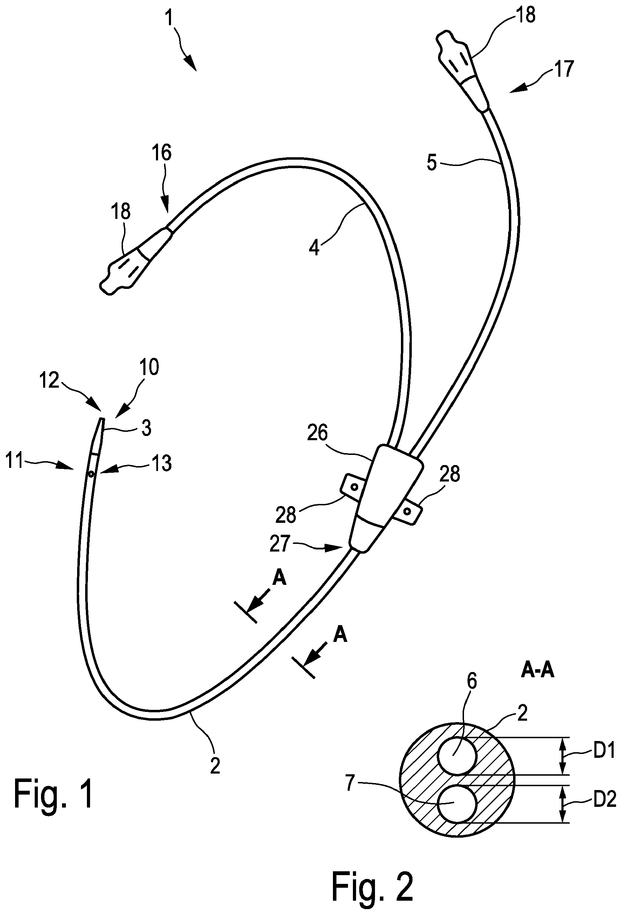

Eine Katheteranordnung 1 nach Fig. 1 ist zur Verwendung bei einer Infusionstherapie vorgesehen. Die Katheteranordnung 1 ist in Gestalt einer zweilumigen Zentralvenenkatheteranordnung ausgebildet. Dessen ungeachtet ist die erfindungsgemäße Lösung selbstverständlich auch bei ein-, drei- oder mehrlumigen Katheteranordnungen anwendbar und nicht auf Zentralvenenkatheteranordnungen beschränkt, sondern insbesondere auch bei einer peripher eingeführten Katheteranordnung oder einer Midline-Katheteranordnung anwendbar.

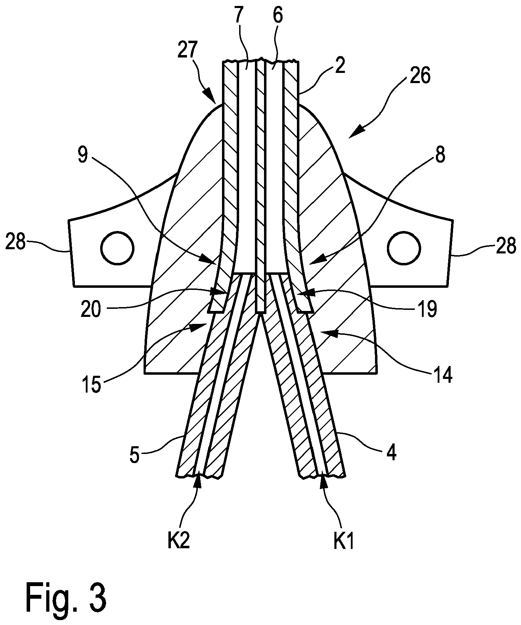

Die Katheteranordnung 1 weist einen Katheterschlauch 2 mit einer Katheterspitze 3 sowie zwei Katheterzuleitungen 4, 5, die nachfolgend als erste Zuleitung 4 und zweite Zuleitung 5 bezeichnet werden, auf. Sowohl der Katheterschlauch 2 als auch die Zuleitungen 4, 5 sind aus Kunststoff gefertigt und biegeflexibel. Wie insbesondere anhand Fig. 2 ersichtlich ist, weist der Katheterschlauch 2 ein erstes Lumen 6 und ein zweites Lumen 7 auf. Die Lumen 6, 7 erstrecken sich voneinander getrennt durch den Katheterschlauch 2 und weisen jeweils ein proximales Ende 8, 9 (Fig. 3) sowie ein distales Ende 10, 11 auf. Das distale Ende 10 des ersten Lumens 6 mündet in eine Austrittsöffnung 12 der Katheterspitze 3. Das distale Ende 11 des zweiten Lumens 7 mündet in eine Radialöffnung 13 des Katheterschlauchs 2. Weiter weisen die Zuleitungen 4, 5 jeweils ein distales Ende 14, 15 (Fig. 3) und ein proximales Ende 16, 17 auf. Sowohl das proximale Ende 16 der ersten Zuleitung 4 als auch das proximale Ende 17 der zweiten Zuleitung 5 ist mit jeweils einem Anschlusselement 18 versehen. Die Anschlusselemente 18 sind jeweils zur fluidleitenden Verbindung der Zuleitungen 4, 5 mit einem nicht näher ersichtlichen Infusionssystem, insbesondere einem Infusionsbehälter oder dergleichen, vorgesehen. Zu diesem Zweck können die Anschlusselemente 18 jeweils mit einem als solchen bekannten Luer-Anschluss versehen sein. Wie anhand Fig. 3 ersichtlich ist, ist das distale Ende 14 der ersten Zuleitung 4 fluidleitend mit dem proximalen Ende 8 des ersten Lumens 6 und das distale Ende 15 der zweiten Zuleitung 5 fluidleitend mit dem proximalen Ende 9 des zweiten Lumens 7 verbunden. Auf diese Weise werden zwei voneinander getrennte Kanäle K1, K2 gebildet. Die Kanäle K1, K2 erstrecken sich ausgehend vom proximalen Ende 16, 17 der jeweiligen Zuleitung 4, 5 bis hin zu dem distalen Ende 10, 11 des der jeweiligen Zuleitung 4, 5 zugeordneten Lumens 6, 7. Dies ermöglicht beispielsweise eine simultane Verabreichung inkompatibler medizinischer Flüssigkeiten mittels der Katheteranordnung 1, wobei eine Vermischung der Flüssigkeiten innerhalb der Katheteranordnung 1 vermieden wird.

Wie weiter insbesondere anhand Fig. 3 ersichtlich ist, sind die fluidleitenden Verbindungen zwischen den Zuleitungen 4, 5 und den Lumen 6, 7 mittels jeweils einer fluiddichten Fügeverbindung 19, 20 zwischen dem distalen Ende 14, 15 der jeweiligen Zuleitung 4, 5 und dem proximalen Ende 8, 9 des jeweiligen Lumens 6, 7 bewirkt. Zu diesem Zweck ist das distale Ende 14 der ersten Zuleitung 4 in das proximale Ende 8 des ersten Lumens 6 eingesteckt und das distale Ende 15 der zweiten Zuleitung 5 ist in das proximale Ende 9 des zweiten Lumens 7 eingesteckt. Vorliegend sind die Steckverbindungen zwischen den Zuleitungen 4, 5 und dem jeweiligen Lumen 6, 7 derart gestaltet, dass die Fügeverbindungen 19, 20 fluiddicht sind. Zu diesem Zweck ist jeweils eine Presspassung zwischen den distalen Enden 14, 15 der Zuleitungen 4, 5 und dem jeweiligen proximalen Ende 8, 9 der Lumen 6, 7 vorgesehen.

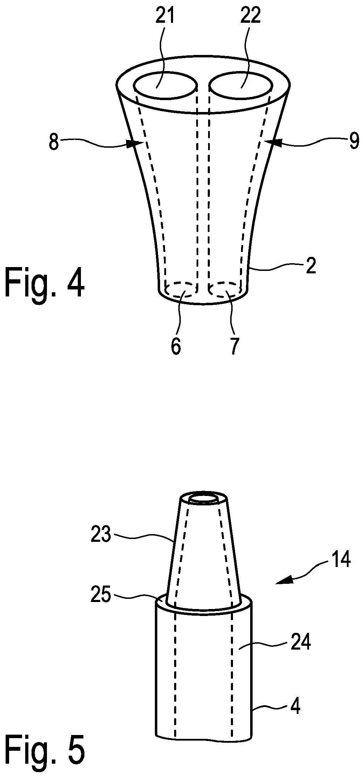

Weitere Einzelheiten der fluiddichten Fügeverbindungen 19, 20 sind insbesondere anhand der Fig. 4 und 5 ersichtlich. Wie anhand Fig. 4 ersichtlich ist, ist an die proximalen Enden 8, 9 der Lumen 6, 7 stirnendseitig jeweils ein Aufnahmeabschnitt 21, 22 angeformt. Im Vergleich mit Fig. 2 ist ersichtlich, dass die Aufnahmeabschnitte 21, 22 gegenüber einem Strömungsquerschnitt D1, D2 des jeweiligen Lumens 6, 7 radial aufgeweitet sind. Zudem sind die distalen Enden 14, 15 der Zuleitungen 4, 5 jeweils mit einem Einführabschnitt versehen. Fig. 5 zeigt die mit einem Einführabschnitt 23 versehene erste Zuleitung 4. Die zweite Zuleitung 5 ist im Bereich ihres distalen Endes 15 in entsprechender Weise ausgestaltet, so dass zur Vermeidung von Wiederholungen anhand Fig. 5 lediglich der Einführabschnitt 23 der ersten Zuleitung 4 näher beschrieben wird. Der Einführabschnitt 23 ist stirnendseitig an das distale Ende 14 der ersten Zuleitung 4 angeformt und komplementär zu dem Aufnahmeabschnitt 21 des proximalen Endes 8 des ersten Lumens 6 gestaltet. Insoweit weist der Einführabschnitt 23 eine konische Außenkontur auf und ist zudem gegenüber einer Mantelfläche 24 der ersten Zuleitung 4 radial abgesetzt, so dass zwischen dem Einführabschnitt 23 und dem weiteren axialen Verlauf der ersten Zuleitung 4 ein Radialbund 25 angeordnet ist. Dieser Radialbund 25 muss jedoch nicht zwingend vorhanden sein. Stattdessen kann der Bereich beispielsweise konisch oder zylindrisch gestaltet sein. Anhand Fig. 3 ist erkennbar, dass das distale Ende 14 der ersten Zuleitung 4 derart in das proximale Ende 9 des ersten Lumens 6 eingesteckt ist, dass der Radialbund 25 stirnendseitig an dem Katheterschlauch 2 zur Anlage gelangt, so dass die Zuleitung 4 in Axialrichtung gegenüber dem Katheterschlauch 2 festgelegt ist. Entsprechendes gilt vorliegend für die Steckverbindung zwischen der zweiten Zuleitung 5 und dem zweiten Lumen 7, was jedoch nicht notwendigerweise der Fall sein muss.

Die Aufnahmeabschnitte 21, 22 sind vorliegend in Form jeweils eines Innenkonus ausgebildet, wobei die Querschnitte der Aufnahmeabschnitte 21, 22 in ihrer Grundform den Strömungsquerschnitten D1, D2 entsprechen und insoweit kreisförmig ausgebildet sind. Selbstverständlich ist es auch möglich, dass eine hiervon abweichende Ausgestaltung der Strömungsquerschnitte und der Aufnahmeabschnitte vorgesehen ist. Beispielsweise können die Strömungsquerschnitte der Lumen oval, halbkreis-, halbmond- oder sichelförmig gestaltet sein. In einem solchen Fall weisen die Aufnahmeabschnitte eine dementsprechend ausgebildete Form auf. Auch die Einführabschnitte der Zuleitungen sind in einem solchen Fall dementsprechend in ihrer Form angepasst.

Vorliegend sind die vorbeschriebenen Steckverbindungen zwischen den Zuleitungen 4, 5 und dem jeweiligen Lumen 6, 7 als solche fluiddicht ausgeführt, so dass sich die Fluiddichtigkeit der Fügeverbindungen 19, 20 bereits durch die Ausgestaltung dieser Steckverbindungen ergibt. Zusätzlich sind die Fügeverbindungen 19, 20 verschweißt. Sofern die Steckverbindungen zwischen den Zuleitungen 4, 5 und dem jeweiligen Lumen 6, 7 nicht bereits als solches fluiddicht ausgeführt sein sollten, kann die Fluiddichtigkeit der Fügeverbindungen 19, 20 auch allein durch ein Verschweißen der distalen Enden 14, 15 der Zuleitungen 4, 5 mit dem proximalen Ende 8, 9 des jeweiligen Lumens 6, 7 bewirkt sein.

Wie weiter anhand Fig. 1 ersichtlich ist, weist die Katheteranordnung 1 zudem eine Kapseleinheit 26 auf. Die Kapseleinheit 26 ist als Niederdruck-Spritzgussbauteil aus Kunststoff gefertigt und derart gestaltet und/oder angeordnet, dass der Bereich der fluiddichten Fügeverbindungen 19, 20 zwischen den Zuleitungen 4, 5 und den Lumen 6, 7 mittels der Kapseleinheit 26 verkapselt ist. Dies ist zudem anhand Fig. 3 ersichtlich. Die Kapseleinheit 26 als solche bildet keinen fluidführenden Abschnitt der Kanäle K1, K2. Stattdessen bewirkt die Kapseleinheit 26 eine zusätzliche Fluidabdichtung der fluiddichten Fügeverbindungen 19, 20. Zudem wird mittels der Kapseleinheit 26 eine Zugkraftentlastung der fluiddichten Fügeverbindungen 19, 20 bewirkt, indem die Kapseleinheit 26 zum einen fest mit den Zuleitungen 4, 5 und zum anderen fest mit dem Katheterschlauch 2 verbunden ist. Die Kapseleinheit 26 ist aus einem Schmelzklebstoff gefertigt. Mittels Niederdruck-Spritzguss verarbeitbare Schmelzklebstoffe sind grundsätzlich bekannt. Beispielsweise kann als Schmelzklebstoff Polyamid oder Polyolefin verwendet werden. Wie weiter anhand Fig. 1 sowie Fig. 3 ersichtlich ist, weist die Kapseleinheit 26 einen Anlageabschnitt 27 auf, der eine im Wesentlichen konische Grundform aufweist. Der Anlageabschnitt 27 ist in einem bestimmungsgemäß applizierten Zustand der Katheteranordnung 1 dazu vorgesehen, an einer patientenseitigen Eintrittsstelle des Katheterschlauchs 2 zur Anlage zu gelangen. Infolge der konischen Ausgestaltung des Anlageabschnitts 27 kann einer Blutung an der vorbeschriebenen Eintrittsstelle des Katheterschlauchs 2 entgegengewirkt werden. Zudem weist die Kapseleinheit 26 zwei Fixierabschnitte 28 auf. Die Fixierabschnitte 28 sind an gegenüberliegenden Seiten der Kapseleinheit 26 angeordnet. Mittels der Fixierabschnitte 28 kann die Kapseleinheit und damit die Katheteranordnung 1 auf grundsätzlich bekannte Weise patientenseitig fixiert werden.

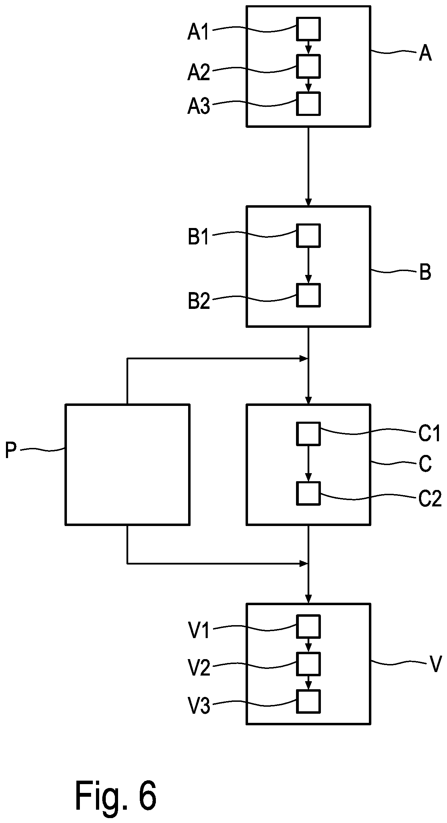

Eine Ausführungsform eines erfindungsgemäßen Verfahrens nach den Fig. 6 bis 9 ist zur Herstellung einer Katheteranordnung 1 nach den Fig. 1 bis 5 vorgesehen. Hierbei geht das Verfahren davon aus, dass sowohl die Zuleitungen 4, 5 als auch der Katheterschlauch 2 in Form grundsätzlich bekannter Kunststoffhalbzeuge vorliegen.

Das Verfahren sieht zunächst einen Schritt vor: (A) Anformen jeweils eines stirnendseitigen Einführabschnitts (23, vgl. Fig. 5) an das distale Ende 14 der ersten Zuleitung 4 und an das distale Ende 15 der zweiten Zuleitung 5.

Hiernach sieht das Verfahren einen weiteren Schritt vor: (B) Anformen jeweils eines stirnendseitigen Aufnahmeabschnitts (21, 22, vgl. Fig. 4) an das proximale Ende 8 des ersten Lumens 6 und an das proximale Ende 9 des zweiten Lumens 7 mittels radialen Aufweitens des ersten Lumens 6 bzw. des zweiten Lumens 7.

Hiernach sieht das Verfahren einen weiteren Schritt vor: (C) Fluiddichtes Zusammenfügen der distalen Enden 14, 15 der Zuleitungen 4, 5 mit dem proximalen Ende 8, 9 des jeweiligen Lumens 6, 7. Bei diesem Schritt wird das distale Ende 14 der ersten Zuleitung 4 fluiddicht mit dem proximalen Ende 8 des ersten Lumens zusammengefügt und das distale Ende 15 der zweiten Zuleitung 5 wird fluiddicht mit dem proximalen Ende 9 des zweiten Lumens 7 zusammengefügt. Sofern die Lumen 6, 7 an ihren proximalen Enden 8, 9 mit jeweils einem Aufnahmeabschnitt 21, 22 und die Zuleitungen 4, 5 an ihren distalen Enden 14, 15 jeweils mit einem Einführabschnitt versehen sind - was jedoch nicht zwingend erforderlich ist - werden bei diesem Schritt die Aufnahmeabschnitte 21, 22 mit dem Einführabschnitt des jeweiligen Lumens 6, 7 fluiddicht zusammengefügt.

Weiter sieht das Verfahren zeitlich vor und/oder nach dem fluiddichten Zusammenfügen (C) einen Schritt vor: (P) Prüfen einer Zuordnung der Einführabschnitte (23, vgl. Fig. 5) zu den Aufnahmeabschnitten 21, 22 und/oder einer Zuordnung der distalen Enden 14, 15 der Zuleitungen 4, 5 zu den proximalen Enden 8, 9 der Lumen 6, 7. Bei diesem Schritt wird geprüft, ob die erste Zuleitung 4 dem ersten Lumen 6 und die zweite Zuleitung 5 dem zweiten Lumen 7 zugeordnet ist. Auf diese Weise wird eine fehlerhafte Zuordnung, die beispielsweise zu einer Beeinträchtigung der Funktion der Katheteranordnung 1 oder zu einer Undichtigkeit der fluiddichten Fügeverbindungen 19, 20 führen kann, vermieden. Der Schritt Prüfen (P) sieht die Verwendung eines grundsätzlich bekannten Verfahrens der computergestützten Bilderkennung und -verarbeitung vor, das auch als maschinelles Sehen oder Bildverstehen bezeichnet wird.

Nach dem fluiddichten Zusammenfügen (C) bzw. gegebenenfalls dem Prüfen (P) sieht das Verfahren einen weiteren Schritt vor: (V) Verkapseln der fluiddichten Fügeverbindungen 19, 20 zwischen den Zuleitungen 4, 5 und den Lumen 6, 7 mittels Niederdruck-Kunststoffspritzgießens, wobei eine Kapseleinheit (26, vgl. Fig. 1, 3) ausgebildet wird.

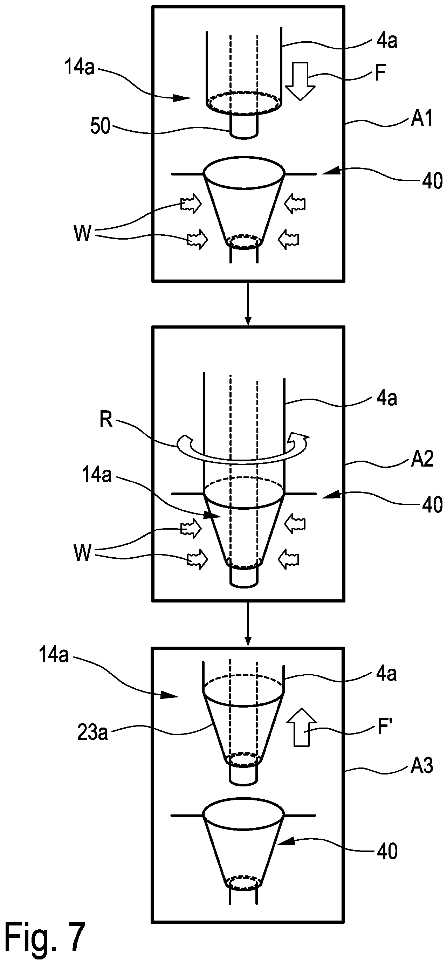

Weitere Details des Verfahrens nach Fig. 6 sind anhand der Fig. 7 bis 9 ersichtlich. Diese beziehen sich auf Einzelheiten der vorbeschriebenen Schritte (A), (B), (C) und (V) des Verfahrens und verdeutlichen diese zudem illustrativ anhand einer weiteren Ausführungsform einer erfindungsgemäßen Katheteranordnung. Diese Katheteranordnung entspricht im Wesentlichen der zuvor anhand der Fig. 1 bis 5 beschriebenen Ausführungsform. Die anhand der Fig. 7 bis 9 jedenfalls bereichsweise ersichtliche Katheteranordnung unterscheidet sich von der Katheteranordnung 1 nach den Fig. 1 bis 5 im Wesentlichen dahingehend, dass diese dreilumig und insoweit mit drei Zuleitungen und drei Lumen anstelle mit zwei Zuleitungen und zwei Lumen (vgl. Fig. 1) versehen ist. Zur Vermeidung von Wiederholungen wird daher auf die Offenbarung zur Ausführungsform nach den Fig. 1 bis 5 verwiesen. Funktions- und/oder baugleiche Abschnitte und Teile der anhand der Fig. 7 bis 9 jedenfalls bereichsweise ersichtlichen Katheteranordnung werden mit gleichen Bezugszeichen unter Hinzufügung des Buchstabens a versehen.

Einzelheiten des Verfahrensschritts Anformen (A) sind anhand Fig. 7 ersichtlich. In einem ersten Teilschritt (A1) wird eine Zuleitung 4a mit ihrem distalen Ende 14a unter Druckeinwirkung - was mittels des Pfeils F verdeutlicht werden soll - in ein Formwerkzeug 40 eingepresst. Das Formwerkzeug 40 weist eine konische Formkontur auf und ist beheizt, was anhand der Pfeile W verdeutlicht werden soll. Das distale Ende 14a ist an seinem Innendurchmesser mittels eines Dorns 50 gestützt. Der Dorn 50 verhindert, dass die Zuleitung 4a beim Einpressen in das Formwerkzeug 40 stirnendseitig verschlossen wird.

In einem weiteren Teilschritt (A2) ist das distale Ende 14a der Zuleitung 4a derart in das Formwerkzeug 40 eingepresst, dass es stirnendseitig am Bodenbereich des Formwerkzeugs 40 zur Anlage gelangt. Zudem wird die Zuleitung 4a um ihre Längsachse rotiert und die Wärmeeinwirkung W aufrechterhalten. Auf diese Weise wird ein Einführabschnitt 23a an dem distalen Ende 14a der Zuleitung 4a ausgebildet.

In einem weiteren Teilschritt (A3) wird die Zuleitung 4a in axialer Richtung aus dem Formwerkzeug 40 entformt. Dies wird anhand des Pfeils F' verdeutlicht.

Je nach Anzahl der vorgesehenen Zuleitungen werden die vorbeschriebenen Teilschritte (A1-A3) für jede der vorgesehenen Zuleitungen wiederholt.

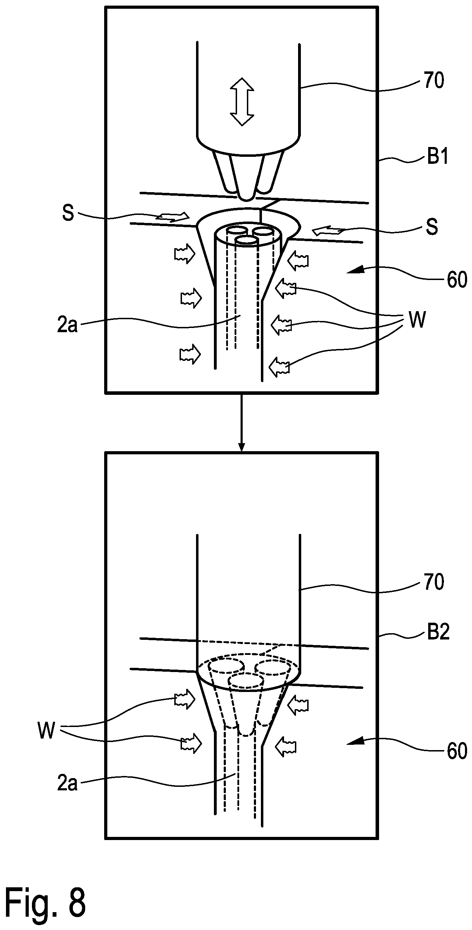

Anhand Fig. 8 sind weitere Einzelheiten des Schritts Anformen (B) des Verfahrens nach Fig. 6 verdeutlicht. Ein erster Teilschritt (B1) sieht vor, dass das proximale Ende eines Katheterschlauchs 2a, der dreilumig ausgestaltet ist, in ein zwei Teilhälften aufweisendes Formwerkzeug 60 gespannt wird. Die Spannbewegung der beiden Teilhälften des Formwerkzeugs 60 soll anhand der Pfeile S verdeutlicht sein. Zudem steht das Formwerkzeug 60 unter Wärmeeinwirkung W. Auf diese Weise wird der Katheterschlauch 2a im Bereich der Einspannung zwischen den beiden Teilhälften erweicht. In einem in Bezug auf die Zeichenebene der Fig. 8 oberen Bereich weist das Formwerkzeug 60 eine konische Aufweitung auf, so dass der Katheterschlauch 2a in diesem Bereich zunächst von dem Formwerkzeug 60 beabstandet ist. Weiter sieht der Teilschritt (B1) vor, dass ein Stempelwerkzeug 70 in axialer Richtung in die proximalen Enden der nicht näher bezeichneten Lumen des Katheterschlauchs 2a eingepresst wird. Das Stempelwerkzeug 70 ist stirnendseitig mit konisch ausgestalteten Zapfen versehen, die beim axialen Verfahren des Stempelwerkzeugs 70 in die proximalen Enden der Lumen eintauchen und diese derart in radialer Richtung aufweiten.

In einem weiteren Teilschritt (B2) ist das Stempelwerkzeug 70 in axialer Richtung vollständig nach unten verfahren, wobei die stirnendseitigen Zapfen des Stempelwerkzeugs 70 vollständig in die proximalen Enden der Lumen des Katheterschlauchs 2a eingetaucht sind. Auf diese Weise werden nicht näher ersichtliche Aufnahmeabschnitte (vgl. Fig. 4, 21, 22) an den proximalen Enden der Lumen ausgebildet.

In einem weiteren nicht näher ersichtlichen Teilschritt wird das Stempelwerkzeug 70 in axialer Richtung nach oben verfahren und der Katheterschlauch 2a wird aus den beiden Teilhälften des Formwerkzeugs 60 ausgespannt und zur weiteren Handhabung aus dem Formwerkzeug 60 entnommen.

Hiernach werden bei dem fluiddichten Zusammenfügen (C) die derart ausgebildeten Einführabschnitte in die Aufnahmeabschnitte der Lumen eingesteckt (C1) und miteinander verschweißt (C2), was schematisch anhand Fig. 6 verdeutlicht ist.

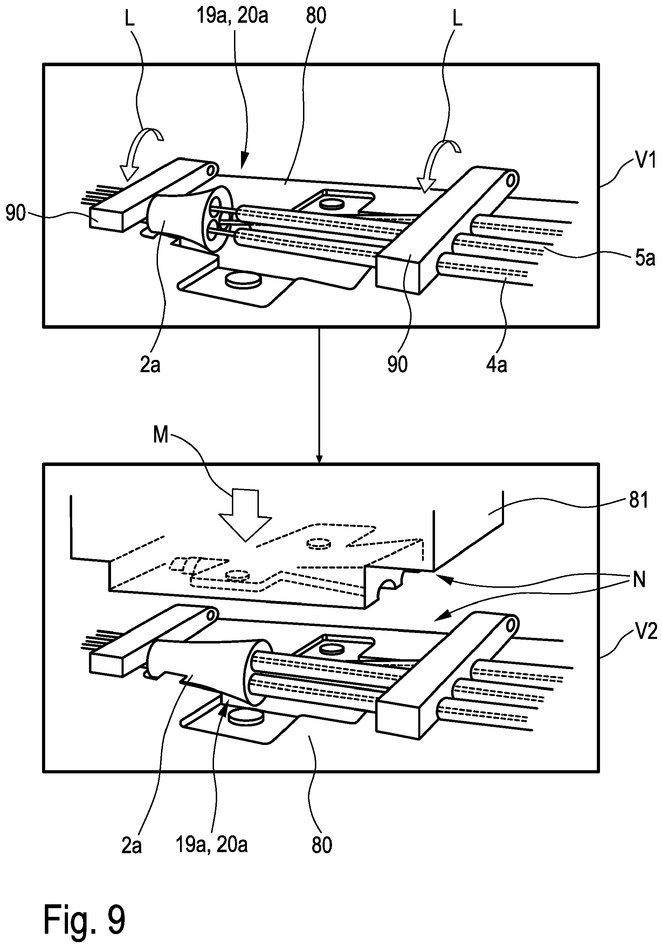

Anhand Fig. 9 sind weitere Einzelheiten des Schritts (V) Verkapseln des Verfahrens nach Fig. 6 ersichtlich. In einem ersten Teilschritt (V1) wird der Katheterschlauch 2a zusammen mit der ersten Zuleitung 4a, einer zweiten Zuleitung 5a und einer weiteren nicht näher bezeichneten dritten Zuleitung in eine untere Teilhälfte 80 eines Niederdruck-Spritzgusswerkzeugs eingelegt. In diesem Zustand sind bereits fluiddichte Fügeverbindungen 19a, 20a zwischen den distalen Enden der jeweiligen Zuleitungen 4a, 5a und dem proximalen Ende des jeweiligen Lumens des Katheterschlauchs 2a ausgebildet. Gleiches gilt für die nicht näher bezeichnete dritte Zuleitung, die ebenfalls fluiddicht mit dem proximalen Ende des ihm zugeordneten Lumens des Katheterschlauchs 2a zusammengefügt ist. Wie anhand Fig. 6 ersichtlich ist, sind die Zuleitungen 4a, 5a zur Ausbildung der fluiddichten Fügeverbindungen 19a, 20a in das jeweilige Lumen des Katheterschlauchs 2a eingesteckt (C1) und/oder mit diesen verschweißt (C2). Die Lage des Katheterschlauchs 2a und der Zuleitungen 4a, 5a und der weiteren nicht näher bezeichneten Zuleitung wird mittels Klemmbacken 90 gegenüber der unteren Teilhälfte 80 des Niederdruck-Spritzgusswerkzeugs fixiert. Dies ist anhand der Pfeile L verdeutlicht.

In einem weiteren Teilschritt (V2) ist vorgesehen, dass eine obere Teilhälfte 81 des Niederdruck-Spritzgusswerkzeugs auf die untere Teilhälfte 80 abgesenkt wird, was anhand des Pfeils M verdeutlicht wird, wobei das Niederdruck-Spritzgusswerkzeug vollständig verschlossen wird. Dabei bilden die Teilhälften 80, 81 eine Negativform N einer nicht näher ersichtlichen Kapseleinheit (vgl. Fig. 1, 26) aus. Nach dem vollständigen Verschließen der beiden Teilhälften 80, 81 wird die Negativform N mit schmelzflüssigem Schmelzklebstoff auf grundsätzlich bekannte Weise bei einer Druckbeaufschlagung in einem Bereich von etwa 3,5 bar - 14 bar befüllt. Auf diese Weise wird eine Kapseleinheit ausgebildet, die die fluiddichten Fügeverbindungen 19a, 20a zwischen den Zuleitungen 4a, 5a und den Lumen des Katheterschlauchs 2a verkapselt.

In einem weiteren Teilschritt (V3) (vgl. Fig. 6) wird die derart gebildete Kapseleinheit auf grundsätzlich bekannte Art und Weise zwischen den Teilhälften 80, 81 des Niederdruck-Spritzgusswerkzeugs entformt, und die Katheteranordnung entnommen.

## Patentansprüche

1. Katheteranordnung (1) mit einem Katheterschlauch (2) mit wenigstens einem ersten Lumen (6, 7), wobei das Lumen (6, 7) ein proximales Ende (8, 9) und ein distales Ende (10, 11) aufweist, und wenigstens einer Zuleitung (4, 5), wobei die Zuleitung (4, 5) ein distales Ende (14, 15) und ein proximales Ende (16, 17) aufweist, und wobei das distale Ende (14, 15) der Zuleitung (4, 5) fluidleitend mit dem proximalen Ende (8, 9) des Lumens (6, 7) verbunden ist, **dadurch gekennzeichnet, dass** die fluidleitende Verbindung zwischen der Zuleitung (4, 5) und dem Lumen (6, 7) mittels einer fluiddichten Fügeverbindung (19, 20) zwischen dem distalen Ende (14, 15) der Zuleitung (4, 5) und dem proximalen Ende (8, 9) des Lumens (6, 7) bewirkt ist.

2. Katheteranordnung (1) nach Anspruch 1, **dadurch gekennzeichnet, dass** das distale Ende (14, 15) der Zuleitung (4, 5) in das proximale Ende (8, 9) des Lumens (6, 7), insbesondere fluiddicht, eingesteckt ist.

3. Katheteranordnung (1) nach Anspruch 1 oder 2, **dadurch gekennzeichnet, dass** das distale Ende (14, 15) der Zuleitung (4, 5) einen stirnendseitig angeformten Einführabschnitt (23) aufweist, der komplementär zu einem stirnendseitig angeformten Aufnahmeabschnitt (21, 22) des proximalen Endes (8, 9) des Lumens (6, 7) ausgebildet ist.

4. Katheteranordnung (1) nach Anspruch 3, **dadurch gekennzeichnet, dass** der Aufnahmeabschnitt (21, 22) gegenüber einem Strömungsquerschnitt (D1, D2) des Lumens (6, 7), insbesondere thermisch, aufgeweitet ist.

5. Katheteranordnung (1) nach einem der vorhergehenden Ansprüche, **dadurch gekennzeichnet, dass** die Fügeverbindung (19, 20), insbesondere fluiddicht, verschweißt ist.

6. Katheteranordnung (1) nach einem der vorhergehenden Ansprüche, **dadurch gekennzeichnet, dass** eine Kapseleinheit (26) vorgesehen ist, die als Niederdruck-Spritzgussbauteil aus Kunststoff gefertigt und derart gestaltet und/oder angeordnet ist, dass der Bereich der fluiddichten Fügeverbindung (19, 20) zwischen der Zuleitung (4, 5) und dem Lumen (6, 7) mittels der Kapseleinheit (26) verkapselt ist, insbesondere wobei die Kapseleinheit (26) eine zusätzliche Fluidabdichtung und/oder eine Zugkraftentlastung der fluiddichten Fügeverbindung (19, 20) bewirkt, und/oder dass die Kapseleinheit (26) aus Schmelzklebstoff, insbesondere aus Polyamid oder Polyolefin, gefertigt ist.

7. Katheteranordnung (1) nach einem der vorhergehenden Ansprüche, **dadurch gekennzeichnet, dass** die Katheteranordnung eine Zentralvenenkatheteranordnung oder eine peripher eingeführte Katheteranordung oder eine Midline-Katheteranordung ist.

8. Verfahren zur Herstellung einer Katheteranordnung nach Anspruch 1 aufweisend einen Schritt: (C) fluiddichtes Zusammenfügen des distalen Endes (14, 15) der Zuleitung (4, 5) mit dem proximalen Ende (8, 9) des Lumens (6, 7).

9. Verfahren nach Anspruch 8 aufweisend einen Schritt: (A) Anformen eines stirnendseitigen Einführabschnitts (23) an das distale Ende (14, 15) der Zuleitung (4, 5).

10. Verfahren nach Anspruch 8 oder 9 aufweisend einen Schritt: (B) Anformen eines stirnendseitigen Aufnahmeabschnitts (21, 22) an das proximale Ende (8, 9) des Lumens (6, 7) mittels radialen Aufweitens des Lumens (6, 7).

11. Verfahren nach Anspruch 9 oder 10, **dadurch gekennzeichnet, dass** das Anformen (A, B) des Einführabschnittes (23) und/oder des Aufnahmeabschnittes (21, 22) mittels Umformen unter Druck- und/oder Wärmeeinwirkung erfolgt.

12. Verfahren nach Anspruch 10 oder 11, **dadurch gekennzeichnet, dass** bei dem fluiddichten Zusammenfügen (C) der Einführabschnitt (23) der Zuleitung (4, 5) in den Aufnahmeabschnitt (21) des Lumens (6, 7) eingesteckt wird (C1).

13. Verfahren nach einem der Ansprüche 8 bis 12, **dadurch gekennzeichnet, dass** bei dem fluiddichten Zusammenfügen (C) das distale Ende (14, 15) der Zuleitung (4, 5) mit dem proximalen Ende (8, 9) des Lumens (6, 7), insbesondere der Einführabschnitt (23) mit dem Aufnahmeabschnitt (21, 22), verschweißt wird (C2).

14. Verfahren nach Anspruch 12 oder 13 aufweisend einen Schritt: (P) Prüfen, insbesondere mittels computergestützter Bilderkennung und -verarbeitung, einer Zuordnung des Einführabschnittes (23) zu dem Aufnahmeabschnitt (21, 22) und/oder einer Zuordnung des distalen Endes (14, 15) der Zuleitung (4, 5) zu dem proximalen Ende (8, 9) des Lumens (6, 7).

15. Verfahren nach einem der Ansprüche 8 bis 14 aufweisend einen Schritt: (V) Verkapseln der fluiddichten Fügeverbindung (19, 20) zwischen der Zuleitung (4, 5) und dem Lumen (6, 7) mittels Niederdruck-Kunststoffspritzgießens, wobei eine Kapseleinheit (26) ausgebildet wird.
